Europäisches Patentamt

European Patent Office

Office européen des brevets

(19)

(11) Numéro de publication : **0 238 400**
**B1**

## (12)  FASCICULE DE BREVET EUROPÉEN

(45) Date de publication du fascicule du brevet :
09.08.89

(51) Int. Cl.⁴ : **C 07 C 15/46, C 07 C 5/333**

(21) Numéro de dépôt : **87400547.3**

(22) Date de dépôt : **12.03.87**

(54) **Procédé de production de styrène.**

(30) Priorité : 21.03.86 FR 8604066

(43) Date de publication de la demande :
**23.09.87 Bulletin 87/39**

(45) Mention de la délivrance du brevet :
**09.08.89 Bulletin 89/32**

(84) Etats contractants désignés :
**AT BE CH DE ES FR GB GR IT LI LU NL SE**

(56) Documents cités :
**DE–A– 1 917 279
US–A– 4 347 396**

(73) Titulaire : **NORSOLOR S.A.
Tour Aurore Place des Reflets
F-92080 Paris la Défense Cédex 5 (FR)**

(72) Inventeur : **Faure, Jacques
Rue des Sablons
F-60550 Verneuil-en-Halatte (FR)**
Inventeur : **Gillet, Philippe
10, Villa Tyrol
F-57500 Saint-Avold (FR)**
Inventeur : **Hess, Raymond
Rue Lepinsecq
F-57600 Forbach (FR)**
Inventeur : **Pognon, Jean-Marc
9, rue des Acacias Tritteling
F-57114 Teting-sur-Nied (FR)**

(74) Mandataire : **Rieux, Michel
c/o NORSOLOR Service Propriété Industrielle B.P. 57
F-62670 Mazingarbe (FR)**

## Description

La présente invention concerne un nouveau procédé de production de styrène par déshydrogénation catalytique de l'éthylbenzène en styrène en présence de vapeur d'eau.

On emploie beaucoup le styrène comme matière première pour la fabrication d'un grand nombre de résines, de matières plastiques et d'élastomères, l'étendue de son emploi étant principalement attribuée à l'aptitude du styrène à se polymériser facilement par exemple en polystyrène, ou à se copolymériser par exemple avec le butadiène pour produire des caoutchoucs.

On connaît bien la production du styrène à la fois en ce qui concerne le procédé de déshydrogénation et les variétés de catalyseurs utilisés dans ce procédé. Actuellement le principal objectif en recherche réside dans l'amélioration de la rentabilité du procédé. Notamment, on connaît un procédé de déshydrogénation d'éthylbenzène (US-A-4347396) selon lequel la déshydrogénation est réalisée dans une installation comportant au moins 3 réacteurs de déshydrogénation en série, des dispositifs de chauffage intermédiaires desdits réacteurs dans lesquels les effluents réactionnels sont soumis à un réchauffage par échange thermique avec la vapeur d'eau. Selon ce procédé, la vapeur d'eau est tout d'abord utilisée pour le réchauffage des effluents réactionnels, et ensuite, elle est mélangée à l'éthylbenzène à l'entrée du premier réacteur. Les conditions opératoires retenues dans ce procédé sont le mélange de 3 à 10 moles de vapeur d'eau à 1 mole d'éthylbenzène, une température et une pression à l'entrée du dernier réacteur respectivement de 600-680 °C et de 3,92-7,84.10⁴ Pa. Les conditions de température et de pression à l'entrée des autres réacteurs sont respectivement de 600-680 °C et de 19,6-3,92.10⁴Pa (absolue), et la vitesse spatiale globale d'éthylbenzène est égale ou supérieure à 0,44 h⁻¹. Par vitesse spatiale horaire de l'éthylbenzène on entend le rapport du débit volumique de l'éthylbenzène sur le volume global de catalyseur. Conformément à ce procédé, on obtient un taux de conversion d'éthylbenzène de l'ordre de 65-75 %, voire supérieure et une sélectivité molaire de l'ordre de 90 %. Toutefois, la mise en œuvre d'un tel procédé conduit, au cours du temps, à la dégradation de la sélectivité en styrène ainsi qu'à celle du taux de conversion en éthylbenzène que l'on peut malgré tout limiter par une augmentation des températures réactionnelles. En effet, au cours du temps, on observe la dégradation progressive du catalyseur et l'apparition de dépôt de carbone sur ce catalyseur. De plus, la présence de points chauds sur la ligne de fabrication (notamment dans les dispositifs de chauffage intermédiaires) provoque des réactions de dégradation thermique produisant des composés lourds et encrassants. On estime que le taux de produits lourds formés est de l'ordre de 17 000 ppm, exprimé par rapport à une tonne d'hydrocarbures effluents, voire supérieure. Il en résulte à terme une augmentation de la perte de charge, une diminution des performances du catalyseur et donc la nécessité d'intervention fréquente pour nettoyage. Dans le but d'éliminer les inconvénients d'un tel procédé, la demanderesse a mis au point un nouveau procédé de déshydrogénation de l'éthylbenzène permettant d'obtenir d'excellents taux de conversion en éthylbenzène, supérieur à 73 %, et une sélectivité supérieure à 93 % molaire.

Plus précisément, l'invention a pour objet un procédé de production de styrène par déshydrogénation catalytique de l'éthylbenzène selon lequel on effectue la déshydrogénation dans une installation comportant 3 réacteurs de déshydrogénation en série, un ou plusieurs dispositifs de chauffages disposés entre les réacteurs dans lesquels les effluents réactionnels sont soumis à un réchauffage par échange thermique avec de la vapeur d'eau, qui est dans une première étape utilisée pour le réchauffage des effluents réactionnels, et dans une seconde étape mélangée à l'éthylbenzène à l'entrée du premier réacteur de déshydrogénation. Le procédé selon l'invention est caractérisé en ce que :

on mélange l'éthylbenzène avec la vapeur d'eau dans un rapport molaire de la vapeur d'eau sur l'éthylbenzène compris entre 5/1 et 13/1,

on chauffe le mélange à l'entrée desdits réacteurs à une température comprise entre 580 et 645 °C,

on maintient une pression moyenne dans le premier réacteur comprise entre 5,88 et 9,8.10⁴Pa en pression absolue et une pression comprise entre 3,92 et 6,86.10⁴Pa en pression absolue dans le deuxième et le troisième réacteur,

on impose une vitesse spatiale globale de l'éthylbenzène liquide comprise entre 0,20 et 0,35 h⁻¹.

Conformément à un mode de réalisation préféré du procédé selon l'invention, on impose une vitesse spatiale globale de l'éthylbenzène liquide comprise entre 0,24 et 0,30 h⁻¹.

De préférence, la température à l'entrée des réacteurs est maintenue entre 595 et 630 °C.

De préférence encore, la pression moyenne dans le premier réacteur est comprise entre 5,88 et 7,84.10⁴Pa pression absolue, la pression moyenne dans le deuxième réacteur est comprise entre 4,9 et 6,86.10⁴Pa en pression absolue ou la pression moyenne dans le troisième réacteur est comprise entre 3,92 et 5,88.10⁴Pa en pression absolue.

De préférence encore, on impose un rapport molaire de la vapeur d'eau sur l'éthylbenzène compris entre 7/1 et 13/1, la conversion d'éthylbenzène et la sélectivité molaire étant les meilleures dans cet intervalle.

Le procédé selon l'invention est applicable avec les catalyseurs classiques utilisés pour la déshydrogénation de l'éthylbenzène. On peut notamment citer les catalyseurs à base d'oxyde de fer et comportant notamment du potassium.

On peut mettre en œuvre n'importe quel réacteur de déshydrogénation de l'éthylbenzène pour la réalisation du procédé selon l'invention. Toutefois, on utilise de préférence des réacteurs radiaux du type de ceux décrits dans le brevet français 2 365 370.

Conformément au procédé selon l'invention qui se trouve dans des conditions de température modérée, de basse pression et de basse vitesse spatiale globale d'éthylbenzène liquide, on parvient à limiter de façon appréciable la formation des produits lourds de dégradation. En effet, selon le procédé selon l'invention, le taux de produits lourds formés n'excède pas 5 000 ppm exprimé par rapport à une tonne d'hydrocarbures effluents.

Conformément au procédé selon l'invention, on obtient des taux de conversion de l'éthylbenzène pouvant atteindre 75 % ou supérieurs, et une sélectivité de l'ordre de 95 % voire supérieure.

D'autre part, la diminution de la formation de produits lourds et encrassants de dégradation permet de conserver ces performances au cours du temps et évite les arrêts fréquents des installations pour nettoyage.

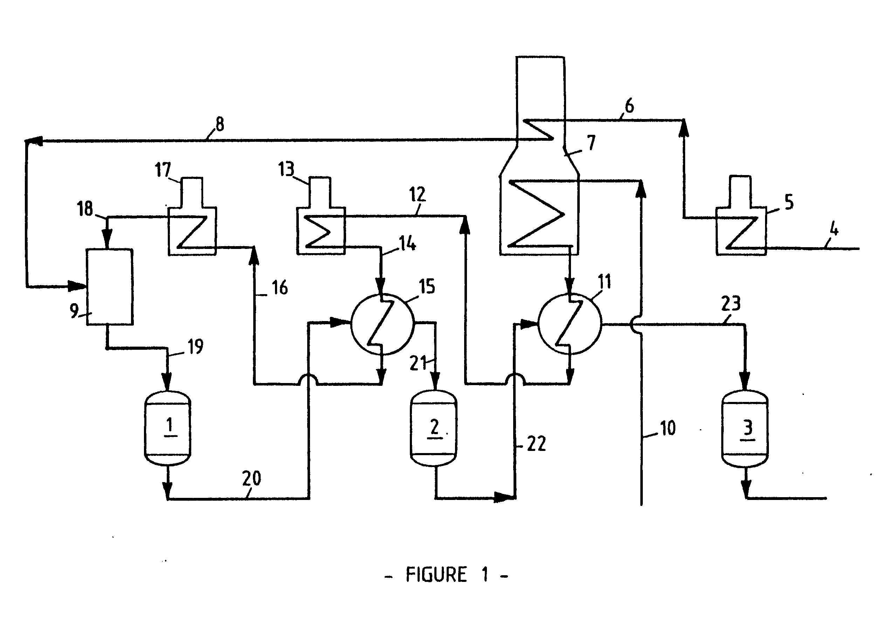

L'invention sera mieux comprise à la lecture des exemples de réalisation pratiques donnés à titre indicatif de la présente invention et réalisés suivant l'installation de fabrication de styrène décrite ci-dessous et représentée sur la figure 1.

Conformément à l'invention, l'installation représentée sur la figure 1 comporte 3 réacteurs de déshydrogénation 1, 2 et 3 disposés en série.

L'éthylbenzène est tout d'abord vaporisé dans un échangeur (non représenté sur la figure 1) puis introduit par le circuit 4 dans un four 5 et ensuite par le circuit 6 dans le four 7. L'éthylbenzène est ensuite amené par le circuit 8 dans le mélangeur 9 pour y être mélangé avec de la vapeur d'eau.

L'eau, qui a été préalablement vaporisée dans une chaudière (non représentée sur la figure 1), est amenée par le circuit 10 dans le four 7. La vapeur d'eau subit ensuite une série d'échanges thermiques, jouant ainsi le rôle de fluide caloporteur à haute température :

à la sortie du four 7, la vapeur d'eau est amenée dans l'échangeur thermique 11 pour réchauffer les effluents issus du réacteur 2,

ensuite, la vapeur d'eau est amenée par le circuit 12 dans le four 13 pour y être réchauffée,

ensuite, la vapeur d'eau est introduite par le circuit 14 dans l'échangeur thermique 15 pour réchauffer les effluents du réacteur 1,

ensuite, la vapeur d'eau est introduite par le circuit 16 dans le four 17 pour y être à nouveau réchauffée,

la vapeur d'eau est ensuite injectée par le circuit 18 dans le mélangeur 9 pour y être mélangée avec l'éthylbenzène.

A l'issue du mélangeur 9, le mélange réactionnel éthylbenzène-vapeur d'eau, dont la fraction molaire de vapeur d'eau sur l'éthylbenzène est, conformément à l'invention, comprise entre 5 et 13, possède une température de l'ordre de 580-645 °C.

Le mélange réactionnel ainsi obtenu est injecté par le circuit 19 dans le réacteur 1 dans lequel il traverse le premier lit catalytique. La pression aux bornes de ce réacteur doit être réglée de manière à avoir une pression moyenne en pression absolue de $5{,}88\text{-}9{,}8.10^4$Pa et de préférence de 5,88 à $7{,}84.10^4$Pa. A sa sortie du réacteur 1 le mélange réactionnel ou effluent est conduit par le circuit 20 dans l'échangeur thermique 15 pour réchauffage. En raison de l'endothermicité de la réaction de déshydrogénation, le mélange subit une baisse de température de quelques dizaines de degrés dans les réacteurs de déshydrogénation 1, 2 et 3. De manière avantageuse et conformément à l'invention, les effluents réactionnels sont réchauffés dans les échangeurs thermiques 15 et 11 avant d'être respectivement injectés dans les réacteurs 2 et 3. A la sortie de l'échangeur 15, le mélange réactionnel possède une température suffisante pour subir à nouveau une déshydrogénation à une température comprise entre 580 et 645 °C. L'effluent est donc conduit par le circuit 21 dans le réacteur 2 dans lequel il traverse le deuxième lit catalytique. La pression aux bornes de ce réacteur doit être réglée de manière à avoir une pression moyenne en pression absolue de préférence de $4{,}9\text{-}6{,}86.10^4$Pa, et de préférence encore de $5{,}1\text{-}6{,}37.10^4$Pa. A la sortie de ce réacteur, l'effluent est amené par le circuit 22 dans l'échangeur thermique 11 pour réchauffage et ensuite par le circuit 23 dans le réacteur 3 dans lequel il traverse le troisième lit catalytique. La pression aux bornes de ce réacteur doit être réglée de manière à avoir une pression moyenne en pression absolue de préférence de $3{,}92\text{-}5{,}88.10^4$Pa, et de préférence encore de $3{,}92\text{-}4{,}9.10^4$Pa.

En sortie de zone réactionnelle, l'effluent est conduit tout d'abord dans des appareils de récupération d'énergie (non représentés sur la figure 1) et sont ensuite envoyés vers la phase de purification du styrène.

La vitesse spatiale de l'éthylbenzène liquide dans les réacteurs 1, 2 et 3 est réglée de manière à avoir une vitesse spatiale globale comprise entre 0,20 et 0,35 $h^{-1}$ et de préférence entre 0,24 et 0,30 $h^{-1}$.

Exemples de réalisation pratique n° 1, 2, 3, 4 et 5 et exemples comparatifs 6 et 7

En faisant appel à une installation dont le schéma de production est représenté sur la figure 1, on a exécuté la déshydrogénation catalytique du mélange éthylbenzène-vapeur d'eau. Les conditions de réactions de déshydrogénation et les résultats sont consignés dans le tableau. Les exemples n° 1, 2, 3, 4 et 5 concernent la mise en œuvre du procédé de déshydrogénation selon l'invention tandis que les exemples comparatifs 6 et 7 correspondent à la mise en œuvre du procédé classique de déshydrogénation dont les conditions opératoires sont rappelées dans la première partie de notre demande.

Par ailleurs, le catalyseur utilisé dans les exemples est à base d'oxyde de fer et comporte notamment du potassium.

(Voir tableau page 5)

4

Tableau I

| | EXEMPLES | | | | | EXEMPLES COMPARATIFS | |
| --- | --- | --- | --- | --- | --- | --- | --- |
| | N° 1 | N° 2 | N° 3 | N° 4 | N° 5 | N° 6 | N° 7 |
| Rapport molaire de vapeur d'eau sur l'éthylbenzène | 12,4 | 9,4 | 8,20 | 7 | 5,3 | 6 | 4 |
| Température (en °C) à l'entrée du<br>- 1er réacteur<br>- 2ème réacteur<br>- 3ème réacteur | 620<br>620<br>620 | 625<br>625<br>625 | 620<br>620<br>620 | 595<br>600<br>600 | 600<br>600<br>600 | 650<br>650<br>650 | 650<br>650<br>650 |
| Vitesse spatiale globale (en $h^{-1}$) | 0,24 | 0,27 | 0,30 | 0,25 | 0,27 | 0,44 | 0,44 |
| Pression moyenne (en $10^4$ Pa )<br>- 1er réacteur<br>- 2ème réacteur<br>- 3ème réacteur | 8,24<br>6,27<br>4,9 | 7,45<br>5,88<br>4,61 | 7,35<br>5,69<br>4,41 | 7,06<br>5,78<br>4,61 | 6,96<br>5,59<br>4,61 | 8,82<br>7,94<br>6,67 | 8,13<br>7,84<br>6,67 |
| Conversion d'éthylbenzène | 80 | 73 | 73,3 | 64,7 | 56,6 | 72,1 | 66,7 |
| Sélectivité (en % molaire) | 95,9 | 94,9 | 93,6 | 93,4 | 94,5 | 90,9 | 89,8 |
| Lourds formés (en ppm) | 1200 | 2400 | 4000 | 900-1000 | 2000 | 16800 | 18000 |

EP 0 238 400 B1

**Revendications**

1. Procédé de production de styrène par déshydrogénation catalytique de l'éthylbenzène selon lequel on effectue la déshydrogénation dans une installation comportant trois réacteurs de déshydrogénation en série, un ou plusieurs dispositifs de chauffage disposés entre les réacteurs et dans lesquels les effluents réactionnels sont soumis à un réchauffage thermique avec de la vapeur d'eau qui est, dans une première étape, utilisée pour le réchauffage des effluents réactionnels, et dans une seconde étape mélangée à l'éthylbenzène à l'entrée du premier réacteur de déshydrogénation, procédé caractérisé en ce que :

on mélange l'éthylbenzène avec la vapeur d'eau dans un rapport molaire de vapeur d'eau sur l'éthylbenzène compris entre 5/1 et 13/1,

on chauffe le mélange à l'entrée desdits réacteurs à une température comprise entre 580 et 645 °C,

on maintient une pression moyenne dans le premier réacteur comprise entre 5,88 et $9,8.10^4$Pa (0,6 et 1 kg/cm$^2$) en pression absolue, et une pression comprise entre 3,92 et $6,86.10^4$Pa (0,4 et 0,7 kg/cm$^2$) en pression absolue dans le deuxième et le troisième réacteur,

on impose une vitesse spatiale globale de l'éthylbenzène liquide comprise entre 0,20 et 0,35 h$^{-1}$.

2. Procédé selon la revendication 1, caractérisé en ce que la vitesse spatiale globale de l'éthylbenzène liquide est comprise entre 0,24 et 0,30 h$^{-1}$.

3. Procédé selon la revendication 1 ou 2, caractérisé en ce que la température à l'entrée des réacteurs est maintenue entre 595 et 630 °C.

4. Procédé selon les revendications 1, 2 ou 3, caractérisé en ce que la pression moyenne dans le premier réacteur est comprise entre 5,88 et $7,84.10^4$Pa (0,6 et 0,8 kg/cm$^2$) en pression absolue.

5. Procédé selon l'une quelconque des revendications 1 à 4, caractérisé en ce que la pression moyenne dans le deuxième réacteur est comprise entre (0,5 et 0,7 kg/cm$^2$) 4,9 et $6,86.10^4$Pa en pression absolue.

6. Procédé selon l'une quelconque des revendications 1 à 5, caractérisé en ce que la pression moyenne dans le deuxième réacteur est comprise entre (0,52 et 0,65 kg/cm$^2$) 5,1 et $6,37.10^4$Pa en pression absolue.

7. Procédé selon l'une quelconque des revendications 1 à 6, caractérisé en ce que la pression moyenne dans le troisième réacteur est comprise entre (0,4 et 0,6 kg/cm$^2$) 3,92 et $5,88.10^4$Pa en pression absolue.

8. Procédé selon l'une quelconque des revendications 1 à 7, caractérisé en ce que la pression moyenne dans le troisième réacteur est comprise entre (0,4 et 0,5 kg/cm$^2$) 3,92 et $4,9.10^4$Pa en pression absolue.

**Claims**

1. Process for producing styrene by the catalytic dehydrogenation of ethylbenzene according to which the dehydrogenation is carried out in a plant which comprises three dehydrogenation reactors in series, one or more heating devices arranged between the reactors and in which the reaction effluents are subjected to a thermal heating (sic) with steam which is, in a first stage, used for heating the reaction effluents, and, in a second stage, mixed with the ethylbenzene at the inlet of the first dehydrogenation reactor, process characterized in that :

ethylbenzene is mixed with steam in a molar ratio steam : ethylbenzene of between 5 : 1 and 13 : 1,

the mixture is heated at the inlet of the said reactors to a temperature of between 580 and 645 °C,

an average pressure in the first reactor of between 5.88 and $9.8 \times 10^4$Pa (0.6 and 1 kg/cm$^2$), expressed as absolute pressure, and a pressure of between 3.92 and $6.86 \times 10^4$Pa (0.4 and 0.7 kg/cm$^2$), expressed as absolute pressure, in

the second and the third reactors are maintained and an overall space velocity of liquid ethylbenzene of between 0.20 and 0.35 h$^{-1}$ is imposed.

2. Process according to Claim 1, characterized in that the overall space velocity of the liquid ethylbenzene is between 0.24 and 0.30 h$^{-1}$.

3. Process according to Claim 1 or 2, characterized in that the temperature at the inlet of the reactors is maintained between 595 and 630 °C.

4. Process according to Claims 1, 2 or 3, characterized in that the average pressure in the first reactor is between 5.88 and $7.84 \times 10^4$Pa (0.6 and 0.8 kg/cm$^2$), expressed as absolute pressure.

5. Process according to any one of Claims 1 to 4, characterized in that the average pressure in the second reactor is between (0.5 and 0.7 kg/cm$^2$) 4.9 and $6.86 \times 10^4$Pa, expressed as absolute pressure.

6. Process according to any one of Claims 1 to 5, characterized in that the average pressure in the second reactor is between (0.52 and 0.65 kg/cm$^2$), 5.1 and $6.37 \times 10^4$Pa, expressed as absolute pressure.

7. Process according to any one of Claims 1 to 6, characterized in that the average pressure in the third reactor is between (0.4 and 0.6 kg/cm$^2$) 3.92 and $5.88 \times 10^4$Pa, expressed as absolute pressure.

8. Process according to any one of Claims 1 to 7, characterized in that the average pressure in the third reactor is between (0.4 and 0.5 kg/cm$^2$) 3.92 and $4.9 \times 10^4$Pa, expressed as absolute pressure.

Patentansprüche

1. Verfahren zur Herstellung von Styrol durch katalytische Dehydrierung von Äthylbenzol, bei welchem man die Dehydrierung in einer Anlage vornimmt, die drei in Serie geschaltete Dehydrierungsreaktoren und eine oder mehrere zwischen den Reaktoren angeordnete Heizeinrichtungen enthält, in denen die aus den Reaktoren austretenden Produkte eines Wiederaufheizungsmittels Wasserdampf unterworfen werden, der in einer ersten Stufe für die Wiederaufheizung der aus den Reaktoren austretenden Produkte verwendet wird und in einer zweiten Stufe dem Äthylbenzol am Eingang des ersten Dehydrierungsreaktors zugemischt wird, dadurch gekennzeichnet, daß man

das Äthylbenzol mit dem Wasserdampf in einem Wasserdampf/Äthylbenzol-Molverhältnis zwischen 5/1 und 13/1 mischt,

die Mischung am Eingang der genannten Reaktoren auf eine Temperatur zwischen 580° und 645 °C erhitzt,

in dem ersten Reaktor einen mittleren Druck zwischen 5,88 und $9,8 \times 10^4$Pa (0,6 und 1 kg/cm$^2$) (Absolutdruck) und in dem zweiten und dritten Reaktor einen Druck zwischen 3,92 und $6,86 \times 10^4$Pa (0,4 und 0,7 kg/cm$^2$) (Absolutdruck) aufrechterhält und

dem flüssigen Äthylenbenzol eine Gesamt-Raumgeschwindigkeit zwischen 0,20 und 0,35 h$^{-1}$ erteilt.

2. Verfahren nach Anspruch 1, dadurch gekennzeichnet, daß die Gesamt-Raumgeschwindigkeit des flüssigen Äthylbenzols zwischen 0,24 und 0,30 h$^{-1}$ beträgt.

3. Verfahren nach Anspruch 1 oder 2, dadurch gekennzeichnet, daß die Temperatur am Eingang der Reaktoren zwischen 595° und 630 °C gehalten wird.

4. Verfahren nach Anspruch 1, 2 oder 3, dadurch gekennzeichnet, daß der mittlere Druck in dem ersten Reaktor zwischen 5,88 und $7,84 \times 10^4$Pa (0,6 und 0,8 kg/cm$^2$) (Absolutdruck) beträgt.

5. Verfahren nach irgendeinem der Ansprüche 1 bis 4, dadurch gekennzeichnet, daß der mittlere Durck in dem zweiten Reaktor zwischen 4,9 und $6,86 \times 10^4$Pa (0,5 und 0,7 kg/cm$^2$) (Absolutdruck) beträgt.

6. Verfahren nach irgendeinem der Ansprüche 1 bis 5, dadurch gekennzeichnet, daß der mittlere Druck in dem zweiten Reaktor zwischen 5,1 und $6,37 \times 10^4$Pa (0,52 und 0,65 kg/cm$^2$) (Absolutdruck) beträgt.

7. Verfahren nach irgendeinem der Ansprüche 1 bis 6, dadurch gekennzeichnet, daß der mittlere Druck in dem dritten Reaktor zwischen 3,92 und $5,88 \times 10^4$Pa (0,4 und 0,6 kg/cm$^2$) (Absolutdruck) beträgt.

8. Verfahren nach irgendeinem der Ansprüche 1 bis 7, dadurch gekennzeichnet, daß der mittlere Druck in dem dritten Reaktor zwischen 3,92 und $4,9 \times 10^4$Pa (0,4 und 0,5 kg/cm$^2$) (Absolutdruck) beträgt.

– FIGURE 1 –